# DEMANDE DE BREVET EUROPEEN

(11) **EP 1 190 703 A1**
(43) Date de publication de la demande: **27.03.2002**
(21) Numéro de dépôt: 01402214.9
(22) Date de dépôt: 23.08.2001
(51) Int. Cl.: A61K 7/48

(54) **Composition à base de glycoglycéride et ses utilisations notamment cosmétiques**

(30) Priorité: 21.09.2000 FR 0012043
(71) Demandeur: L'OREAL, 75008 Paris (FR)
(72) Inventeur: Aubrun-Sonneville, Odile, 92160 Antony (FR)
(74) Mandataire: Rasson, Catherine

(57) **Abrégé**

L'invention se rapporte à une composition à usage topique comprenant au moins un glycoglycéride et au moins un agent séquestrant. Le glycoglycéride présente une très bonne stabilité dans la composition de l'invention.

Le glycoglycéride est de préférence un galactolipide tel que le digalactosyl diglycéride ou le monogalactosyl diglycéride. L'agent séquestrant peut être choisi notamment parmi les dérivés phosphoniques et leurs sels.

Cette composition peut constituer en particulier une composition cosmétique et/ou dermatologique.

## Description

L'invention se rapporte à une composition à usage topique stable comprenant au moins un glycoglycéride et au moins un agent séquestrant, et à ses utilisations, en particulier dans les domaines cosmétique et dermatologique.

Il est connu d'utiliser les glycoglycérides naturels et notamment parmi ceux-ci les galactolipides, dans les compositions cosmétiques ou dermatologiques. Ainsi, les documents EP-A-9842 et EP-A-249561 décrivent l'utilisation de galactolipides pour la préparation de liposomes, et le document WO-A-95/20945 décrit l'utilisation de di-galactosyl diglycérides en association avec des lipides non polaires tels que le triacylglycérol, pour la préparation de supports lipophiles utilisables dans les domaines de la pharmacie, la cosmétique et l'agroalimentaire.

Toutefois, les compositions contenant des glycoglycérides naturels présentent l'inconvénient d'évoluer dans le temps, cette évolution se traduisant par le jaunissement de la composition et une odeur de rance très marqués. Ces changements de couleur et d'odeur sont rédhibitoires pour l'utilisateur.

On a essayé de pallier cet inconvénient en ajoutant aux compositions contenant un glycoglycéride naturel, des anti-oxydants classiquement utilisés pour stabiliser les lipides, tels que le dibutylhydroxytoluène (BHT) ou l'acétate de tocophérol. Toutefois, l'ajout de tels anti-oxydants n'a pas résolu les problèmes de jaunissement et de rancissement de ces compositions.

Il substitue donc le besoin d'une composition de glycoglycérides, qui soit stable dans le temps et qui en particulier ne jaunisse ni ne rancisse au cours du temps.

La demanderesse a maintenant trouvé de façon surprenante que les agents séquestrants permettaient de stabiliser les compositions contenant les glycoglycérides et d'éviter notamment qu'elles ne rancissent et ne deviennent jaunes. Si l'utilisation de séquestrants est courante en complément d'un mélange d'anti-oxydants pour empêcher l'oxydation de lipides et pour améliorer l'efficacité de ces derniers, il n'était pas évident que la seule utilisation d'un séquestrant ou d'un mélange de ceux ci, en absence d'anti-oxydants, bloque le jaunissement de glycoglycérides, l'oxydation de la partie lipidique n'étant pas la cause première du jaunissement puisque l'on ne détecte pas de pentane, marqueur d'une oxydation des lipides insaturés.

La présente invention a donc pour objet une composition à usage topique comprenant dans un milieu physiologiquement acceptable, au moins un glycoglycéride et au moins un agent séquestrant.

Elle a aussi pour objet l'utilisation d'au moins un agent séquestrant pour stabiliser une composition à usage topique contenant au moins un glycoglycéride.

Par « composition à usage topique », on entend toute composition pouvant être appliquée sur les matières kératiniques telles que la peau y compris le cuir chevelu, les cheveux, les cils, les ongles et les muqueuses telles que les lèvres.

Par « agent séquestrant », on entend dans la présente invention un composé capable de se combiner aux métaux de transition.

Parmi les agents séquestrants utilisables dans la composition de l'invention, on peut citer par exemple l'acide éthylène diamine tétracétique (EDTA) et ses sels tels que le sel disodique d'éthylène diamine tétracétique (Disodium EDTA) ; les dérivés phosphoniques tels que l'acide hexaméthylène diamine tétra(méthylène phosphonique), l'acide éthylène diamine tétra(méthylène phosphonique), l'acide 1-hydroxyéthylidène 1,1-diphosphonique, l'acide aminotri(méthylène phosphonique), l'acide diéthylène-triamine penta(méthylène phosphonique), et leurs sels et notamment leurs sels de sodium comme le sel pentasodique de l'acide éthylènediamine tétra(méthylène phosphonique) ; les polymères notamment polyaminés tels que les polyalkylène polyamines et leurs dérivés, et en particulier la polyéthylène imine, et les dendrimères à activité chélatante ; les protéines comme la spermine, la spermidine, la transférine, la ferritine ; les acides carboxyliques comme l'acide phytique, l'acide citrique, l'acide malique, l'acide nitrilo-acétique, l'acide fumarique, l'acide tartrique, l'acide succinique, l'acide oxalique ; la desferrioxamine mesylate ; et les mélanges de ces séquestrants.

De manière préférée, l'agent séquestrant est choisi parmi l'acide hexaméthylène diamine tétra(méthylène phosphonique), l'acide éthylène diamine tétra(méthylène phosphonique), l'acide 1-hydroxyéthylidène 1,1-diphosphonique, l'acide aminotri(méthylène phosphonique), l'acide diéthylène-triamine penta(méthylène phosphonique), leurs sels et notamment leurs sels de sodium, et leurs mélanges.

Par exemple, on peut utiliser de manière avantageuse l'acide éthylènediamine tétra(méthylène phosphonique), vendu par la Société Monsanto sous la dénomination Dequest 2041 et le sel pentasodique de ce dernier qui est vendu sous la dénomination Dequest 2046 par la Société Monsanto.

Selon un mode de réalisation de l'invention, la composition selon l'invention peut être exempte d'anti-oxydants.

Les glycoglycérides utilisés dans la composition de l'invention sont des esters comportant une ou deux chaînes alkyle et/ou alkényle, et ils peuvent être obtenus par extraction ou par synthèse. Ceux obtenus par extraction sont notamment extraits de végétaux. Les produits de synthèse peuvent être obtenus en particulier par estérification d'un sucre comportant au moins une fonction acide carboxylique avec un monoglycéride ou un diglycéride. Les sucres concernés sont les oses (monosaccharides), oligoholosides (oligosaccharides) et les polyholosides (polysaccharides) fortement dépolymérisés dont la masse moléculaire est préférentiellement inférieure à 2000 g/mol. A titre d'exemples non limitatifs de ces sucres, on peut citer le glucose, le sucrose, le tréhalose, le sorbitol, le lactose, le fructose, le xylose, le maltose.

Les chaînes alkyle et/ou alkényle du glycoglycéride comportent de préférence de 10 à 22 atomes de carbone et sont choisies par exemple parmi les radicaux caprique, laurique, myristique, palmitique, stéarique, arachidique, béhénique, palmitoléique, oléique, linoléique, linolénique, gadoléique, arachidonique, érucique.

Selon un mode préféré de réalisation de l'invention, les glycoglycérides utilisés sont les galactolipides, en particulier le digalactosyl diglycéride ou 1-O-[6-O-(α-D-galactopyranosyl)-β-D-galacto-pyranosyl]-2,3-di-O-acyl-D-glyceritol (DGDG) qui peut être sous forme hydrogénée ou non hydrogénée ; le monogalactosyl diglycéride (MGDG) et leurs mélanges. Ces composés existent à l'état naturel dans les végétaux et plus particulièrement dans les membranes des végétaux. Ils peuvent être obtenus par extraction de ces végétaux, et par exemple à partir des céréales ou des épinards. Ils sont décrits par exemple dans les documents EP-A-9842 et EP-A-249561 incorporés ici pour référence. On peut utiliser notamment le DGDG et le MGDG fournis par les sociétés Serdary Research Laboratories ou Doosan. On peut aussi préparer le DGDG à partir de feuilles d'épinards fraîches, selon le procédé de l'exemple 1 du document EP-A-249561, ou bien de céréales selon le procédé décrit dans le document EP-A-9842.

Le DGDG a la structure (I) suivante : 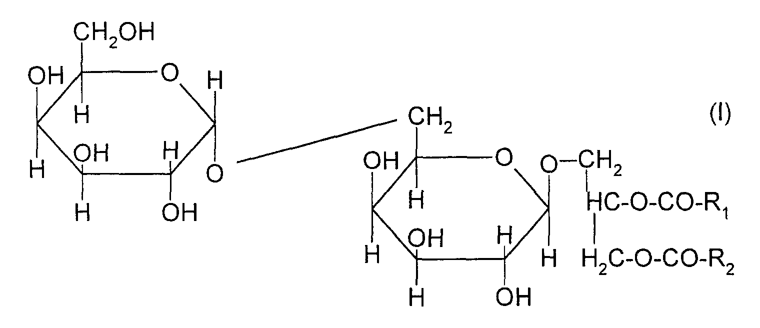 dans laquelle R₁ et R₂ peuvent être identiques ou différents et représentent un reste alkyle et/ou alkényl comportant de 10 à 22 atomes de carbone, tels que les radicaux caprique, laurique, myristique, palmitique, stéarique, arachidique, béhénique, palmitoléique, oléique, linoléique, linolénique, gadoléique, arachidonique, érucique.

On peut utiliser ces galactolipides purs ou dans des mélanges les contenant en des proportions plus ou moins importantes. On utilise de préférence des mélanges contenant au moins 10 % de DGDG par rapport aux lipides polaires du dit mélange.

La quantité d'agent(s) séquestrant(s) varie selon la quantité de glycoglycéride(s) présent(s). Elle peut aller par exemple de 0,01 à 20 % et de préférence de 0,5 à 10 % du poids total de l'association agent séquestrant et glycoglycéride, le ou les glycoglycérides représentant le complément à 100 %. Le rapport pondéral de l'agent séquestrant au glycoglycéride (en matière active) va de préférence de 1.10⁻⁴ à 0,2 et mieux de 0,5.10⁻² à 0,1.

La composition selon l'invention comprenant l'association d'un glycoglycéride et d'un agent séquestrant constitue une composition à usage topique et en particulier une composition cosmétique et/ou dermatologique, et elle comprend un milieu physiologiquement acceptable. On entend par milieu physiologiquement acceptable dans la composition de l'invention, un milieu non toxique et susceptible d'être appliqué sur la peau (y compris l'intérieur des paupières), les cheveux, les ongles ou les lèvres d'êtres humains.

Dans la composition selon l'invention destinée à une application topique, la quantité de glycoglycéride(s) peut aller par exemple de 0,1 à 10 % en poids (en matière active) et de préférence de 0,5 à 5 % en poids par rapport au poids total de la composition, et la quantité d'agent séquestrant(s) peut aller par exemple de 0,001 à 5 % en poids et de préférence de 0,01 à 2 % en poids par rapport au poids total de la composition.

La composition de l'invention comprenant l'association de glycoglycéride et de séquestrant peut se présenter sous toutes les formes galéniques normalement utilisées dans les domaines cosmétique et dermatologique, et elle peut être notamment sous forme d'une dispersion du type lotion éventuellement biphasée, d'une émulsion huile-dans-eau (H/E) ou eau-dans-huile (E/H), ou d'une émulsion triple (E/H/E ou H/E/H) ou d'une dispersion vésiculaire de type ionique (liposomes) et/ou non ionique. Ces compositions sont préparées selon les méthodes usuelles.

La composition selon l'invention peut comprendre une phase huileuse (ou phase grasse) à base d'une ou plusieurs huiles choisies parmi les huiles d'origine végétale (jojoba, avocat, sésame, tournesol, maïs, soja, carthame, pépins de raisin), les huiles minérales (vaseline, isoparaffines hydrogénées ou non hydrogénées, isohexadecane, squalane), les huiles de synthèse (parléam, myristate d'isopropyle, octanoate de cétéaryle, polyisobutylène, palmitate d'éthylhexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles. La phase huileuse peut contenir, en outre d'autres constituants gras tels que les alcools gras comme l'alcool stéarylique, l'alcool cétylique et leur mélange (alcool cétéarylique) ; les acides gras ; les cires ; les gommes de silicone.

La composition selon l'invention peut comprendre une phase aqueuse qui peut contenir, outre l'eau, les solvants et les additifs hydrosolubles ou hydrodispersibles. Comme solvants, on peut citer par exemple les mono-alcools linéaires ou ramifiés ayant de 1 à 8 atomes de carbone comme l'éthanol, le propanol, l'isopropanol, le butanol, l'isobutanol ; les polyéthylène glycols ayant de 4 à 80 oxydes d'éthylène ; les polyols comme la glycérine, le propylène glycol, l'isoprène glycol et le butylène glycol.

Selon un mode particulier de réalisation de l'invention, la composition de l'invention est sous forme d'une émulsion E/H. La phase aqueuse de l'émulsion E/H peut varier dans une large mesure et elle peut constituer jusqu'à 98 % du poids total de la composition. Elle représente généralement de 50 à 90 % en poids par rapport au poids total de la composition, et la phase huileuse représente généralement de 0,5 à 40 % en poids par rapport au poids total de la composition.

Les glycoglycérides sont de préférence introduits dans la phase huileuse de la composition les contenant tandis que l'agent séquestrant est généralement introduit dans la phase aqueuse.

Les compositions de l'invention sous forme d'émulsions peuvent contenir des tensioactifs utilisés habituellement pour la préparation des émulsions. Comme tensioactifs utilisables pour la préparation des émulsions E/H, on peut citer par exemple les alkyl esters ou éthers de sorbitane, de glycérol ou de sucres, les alkyldiméthicone copolyols.

Lorsque la composition de l'invention contient des tensioactifs autres que les glycoglycérides, ces tensioactifs sont présents en une quantité allant de 0,1 à 50 % en poids par rapport au poids de glycoglycérides ou du mélange de lipides contenant les glycoglycérides.

La composition selon l'invention trouve son application dans un grand nombre de traitements notamment cosmétiques de la peau, y compris du cuir chevelu, des cheveux, des ongles, et/ou des muqueuses. en particulier pour le soin, le nettoyage et/ou le maquillage et/ou la protection solaire de la peau, des cheveux et/ou des lèvres ou des muqueuses.

Aussi, la présente invention a pour objet l'utilisation cosmétique d'une composition cosmétique telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition cosmétique telle que définie ci-dessus.

De façon connue, la composition de l'invention peut contenir également des adjuvants habituels dans les domaines cosmétique et/ou dermatologique, tels que les électrolytes comme le sulfate de magnésium, les actifs, les conservateurs, les antioxydants, les solvants, les agents de textures et gélifiants hydrophiles ou lipophiles, les parfums, les charges et notamment les particules minérales ou organiques, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse. En outre, ces adjuvants ainsi que leurs concentrations doivent être tels qu'ils ne modifient pas la propriété recherchée pour la composition de l'invention.

Comme actifs, on peut citer notamment, outre ceux indiqués plus haut, les hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines ; l'urée ; la caféine ; les dépigmentants tels que l'acide kojique et l'acide caféique ; les bêta-hydroxyacides tels que l'acide salicylique et ses dérivés ; les alpha-hydroxyacides tels que l'acide lactique et l'acide glycolique ; les rétinoïdes tels que les caroténoïdes ; les filtres ; et leurs mélanges.

Le ou les actifs peuvent être par exemple présents en une concentration allant de 0,01 à 20 %, de préférence de 0,1 à 5 % et mieux de 0,5 à 3 % du poids total de la composition.

L'exemple ci-après de composition selon l'invention est donné à titre d'illustration et sans caractère limitatif. Les quantités y sont données en % en poids, sauf mention contraire.

| **Exemple selon l'invention : Emulsion E/H** | |
|---|---|
| *A*. *Phase huileuse* | |
| Galactolipides (DGDG fourni par la société Doosan) | 2,5 % |
| Isohexadécane | 7,7 % |
| Squalane | 5,8 % |
| Cyclométhicone | 3,9 % |
| Conservateurs | 0,1 % |

| *B*. *Phase aqueuse* | |
|---|---|
| MgSO₄.7H₂O | 0,8 % |
| Conservateurs | 0,6 % |
| Parfum | 0,2 % |
| Dequest 2046 | 0,1 % |
| Eau | qsp 100 % |

On obtient une crème fluide stable au moins deux mois à 45°C.

| **Exemple comparatif : Emulsion E/H** | |
|---|---|
| *A*. *Phase huileuse* | |
| Galactolipides (DGDG fourni par la société Doosan) | 2,5 % |
| Isohexadécane | 7,7 % |
| Squalane | 5,8 % |
| Cyclométhicone | 3,9 % |
| Conservateurs | 0,1 % |
| BHT | 0,07 % |
| Acétate de tocophérol | 0,75 % |

| *B*. *Phase aqueuse* | |
|---|---|
| MgSO₄.7H₂O | 0,8 % |
| Conservateurs | 0,6 % |
| Parfum | 0,2 % |
| Eau | qsp 100 % |

Le BHT et l'acétate de tocophérol sont des anti-oxydants.

### Test comparatif :

On a mesuré au chromamètre le jaunissement respectif les compositions de l'exemple selon l'invention et de l'exemple comparatif après un stockage de deux mois à 45°C. La mesure a été effectuée directement sur le flacon contenant la composition à l'aide d'un chromamètre CR-300 de la société Minolta (paramètres techniques : géométrie de mesure : d/0°, taille de la zone de mesure : 8 mm, source : lampe à arc xénon, illuminant D65). La couleur mesurée est caractérisée par 3 coordonnées (L, a, b) dans l'espace L*a*b dans lequel L représente la clarté, a représente l'axe rouge-vert (une valeur négative de a correspond au vert, et une valeur positive de a correspond au rouge) et b représente l'axe jaune-bleu (une valeur négative de b correspond au bleu, et une valeur positive de b correspond au jaune). Et si a=b=0 la teinte est grise.

Par ailleurs, l'odeur de rance est appréciée par le chimiste ayant préparé la composition, ainsi que par un expert en parfums.

| | Exemple selon l'invention | | Exemple comparatif | |
|---|---|---|---|---|
| | Temps zéro | Après deux mois à 45°C | Temps zéro | Après deux mois à 45°C |
| L | 77,72 | 76,16 | 81,02 | 81,49 |
| a | -0,51 | -0,64 | -1,28 | -2,26 |
| b | +3,29 | +3,32 | +4,55 | +8,67 |
| Odeur | aucune | aucune | aucune | forte |

Ce tableau montre que, pour l'exemple comparatif, les composantes verte et bleue augmentent très significativement au bout du temps de conservation, induisant de fait une augmentation de la couleur jaune d'ensemble. De plus, une forte odeur de rance, significative de la dégradation du galactolipide, s'est développée. Celle-ci est rédhibitoire pour la commercialisation d'une telle composition. En revanche, la composition selon l'invention ne présente pas d'odeur de rance, et sa couleur est inchangée : ses caractéristiques chromatiques n'ont pas évolué et sa couleur est restée parfaitement stable.

## Revendications

1. Composition à usage topique comprenant dans un milieu physiologiquement acceptable, au moins un glycoglycéride et au moins un agent séquestrant.

2. Composition selon la revendication 1, **caractérisée en ce que** l'agent séquestrant est choisi parmi l'acide éthylène diamine tétracétique (EDTA) et ses sels ; les dérivés phosphoniques et leurs sels ; les polymères polyaminés et leurs dérivés ; les protéines ; les acides carboxyliques ; la desferrioxamine mesylate ; et leurs mélanges.

3. Composition selon la revendication 1 ou 2, **caractérisée en ce que** l'agent séquestrant est choisi parmi l'acide hexaméthylène diamine tétra(méthylène phosphonique), l'acide éthylène diamine tétra(méthylène phosphonique), l'acide 1-hydroxyéthylidène 1,1-diphosphonique, l'acide aminotri(méthylène phosphonique), l'acide diéthylène-triamine penta(méthylène phosphonique), leurs sels, et leurs mélanges.

4. Composition selon l'une quelconque des revendications précédentes, **caractérisée en ce que** le glycoglycéride est obtenu par synthèse ou par extraction.

5. Composition selon l'une quelconque des revendications précédentes, **caractérisée en ce que** le glycoglycéride est extrait d'un végétal.

6. Composition selon l'une quelconque des revendications 1 à 4, **caractérisée en ce que** le glycoglycéride est obtenu par estérification d'un sucre comportant au moins une fonction acide carboxylique avec un monoglycéride ou un diglycéride.

7. Composition selon l'une quelconque des revendications précédentes, **caractérisée en ce que** le glycoglycéride est un galactolipide.

8. Composition selon la revendication précédente, **caractérisée en ce que** le galactolipide est choisi parmi le digalactosyl diglycéride hydrogéné ou non hydrogéné, le monogalactosyl diglycéride et leurs mélanges.

9. Composition selon l'une quelconque des revendications précédentes, **caractérisée en ce que** le rapport pondéral de l'agent séquestrant au glycoglycéride va de 1.10⁻⁴ à 0,2.

10. Composition selon l'une quelconque des revendications précédentes, **caractérisée en ce que** la quantité de glycoglycéride(s) va de 0,1 à 10 % en poids par rapport au poids total de la composition en poids par rapport au poids total de la composition.

11. Composition selon l'une quelconque des revendications précédentes, **caractérisée en ce que** la quantité d'agent séquestrant(s) va de 0,001 à 5 % en poids par rapport au poids total de la composition.

12. Composition selon l'une quelconque des revendications précédentes, **caractérisée en ce qu'**elle se présente sous forme d'une dispersion, d'une émulsion huile-dans-eau ou eau-dans-huile, d'une émulsion triple ou d'une dispersion vésiculaire de type ionique et/ou non ionique.

13. Composition selon l'une quelconque des revendications précédentes, **caractérisée en ce qu'**elle se présente sous forme d'une émulsion eau-dans-huile.

14. Utilisation cosmétique d'une composition cosmétique selon l'une quelconque des revendications précédentes, pour le traitement, la protection, le soin, le démaquillage et/ou le nettoyage de la peau, des lèvres et/ou des cheveux, et/ou pour le maquillage de la peau et/ou des lèvres.

15. Procédé de traitement cosmétique de la peau, y compris du cuir chevelu, des cheveux, et/ou des lèvres, **caractérisé en ce que** l'on applique sur la peau, les cheveux et/ou les lèvres, une composition cosmétique selon l'une quelconque des revendications 1 à 13.

16. Utilisation d'au moins un agent séquestrant pour stabiliser une composition à usage topique contenant au moins un glycoglycéride.
